Europäisches Patentamt

European Patent Office

Office européen des brevets

(19)

(11) Veröffentlichungsnummer: **O OOO O68**
**B1**

(12) **EUROPÄISCHE PATENTSCHRIFT**

(45) Veröffentlichungstag der Patentschrift: **07.04.82**

(21) Anmeldenummer: **78100126.8**

(22) Anmeldetag: **12.06.78**

(51) Int. Cl.³: **A 61 B  10/00,**
**G 01 S  15/42, G 10 K  11/34**

(54) Vorrichtung zur Ultraschalldarstellung mittels dynamischer Fokussierung.

(30) Priorität: **13.06.77 US  806005**

(43) Veröffentlichungstag der Anmeldung:
**20.12.78 Patentblatt 78/1**

(45) Bekanntmachung des Hinweises auf die Patenterteilung:
**07.04.82 Patentblatt 82/14**

(84) Benannte Vertragsstaaten:
**BE CH DE FR GB NL SE**

(56) Entgegenhaltungen:
**GB - A - 820 165**
**NL - A - 77 12029**
**US - A - 3 090 030**
**US - A - 3 598 559**
**US - A - 4 011 750**
**US - A - 4 012 952**
**US - A - 4 019 169**
**US - A - 4 058 003**
**US - A - 4 084 582**

(73) Patentinhaber: **NEW YORK INSTITUTE OF**
**TECHNOLOGY**
**Wheatley Road**
**Old Westbury, New York 11 568 (US)**

(72) Erfinder: **Glenn, William E., Dr.**
**650 Royal Plaza, Fort Lauderdale**
**Florida 33301 (US)**

(74) Vertreter: **Daum, Martin, Dr. et al,**
**Boehringer Mannheim GmbH. Sandhoferstrasse**
**116**
**D-6800 Mannheim 31 (DE)**

(56) Entgegenhaltungen:
**Quarterly Progress Report No. 98, (1970)**
**Research Laboratory for Electronics**
**Massachusetts Institute of technology,**
**Cambridge Mass. (US), Hubelbank & Tretiak**
**"Focused ultrasonic transducer design", Seiten**
**169—177.**

CCD 75 Proceeding of CCD Applications Conference Naval Electronics Laboratory Center, 29—31 Oktober '75, San Diego California, Herausgeber: Learned Information, New York (US) und Oxford (GB Melen u.a. "CCD dynamically focussed lenses for ultrasonic imaging systems" Seiten 165—171.

Electronics And Communications In Japan Band 58-A Nr. 12, Dezember 1975, Washington DC (US), UEDA u.a. "Dynamic focusing ultrasonic transducers using analog-switch phase shifters", Seiten 1—8.

## Vorrichtung zur Ultraschalldarstellung mittels dynamischer Fokussierung

Die Erfindung betrifft eine Vorrichtung zur bildlichen Darstellung eines Körpers mit einer Sendeeinrichtung zum Austrahlen von Energie in den Körper, einem Wandler zur Umwandlung von aus dem Körper reflektierter Energie in elektrische Signale, der in eine Mehrzahl getrennter Elemente unterteilt ist, einer Mehrzahl von Speicherregistern wobei der Eingang jedes Speicherregisters mit einem zugeordneten Wandlerelement verbunden ist und einer Summierungsschaltung zum Zusammenfassen der aus den Speicherregistern ausgelesenen Information, um ein Bildsignal zu erhalten.

Während der letzten zwei Jahrzehnte ist die Ultraschalltechnik in der klinischen Diagnostik stets bedeutungsvoller geworden. Die Ultraschalltechnik wurde unter anderem bereits im Bereich der Gynäkologie, der Neurologie und der Kardiologie verwendet, wobei sie z.B. bei der Sichtbarmachung sabkutaner Blutgefäße (einschließlich kleinerer Gefäße) erfolgreich angewandt wurde.

Für die Anwendung der Ultraschalltechnik in der Medizin sprechen bedeutende Gründe: Ultraschall unterscheidet sich von anderer Art von Bestrahlung durch die damit verbundene harmlose Auswirkung auf lebende Systeme, weil sie rein mechanischer Wellennatur ist. Durch die Ultraschalltechnik ist Information erreichbar, die von anderen Methoden, beispielsweise durch Untersuchung mit $\gamma$- und Röntgenstrahlen, nicht erreichbar ist. Vor allem ist das Risiko einer Verletzung bei der Verwendung von Ultraschall viel geringer als z.B. bei der Verwendung ionisierender Strahlen ($\gamma$- oder Röntgenstrahlen). Ultraschall wird in der Hauptsache als Pulsechomethode in der diagnostischen Technik verwendet, wozu Impulse von Ultraschallenergie periodisch von einem piezoelektrischen Geber, z.B. auf Blei-Zirkonat-Titanat Keramik-Basis, erzeugt werden. Jeder kleine Impuls Ultraschallenergie wird als Schallwelle gebündelt auf den Körper des Patienten gerichtet, wobei er über gegebenenfalls verschiedene Strukturen der Oberfläche eindringt. Hat eine Grenzfläche des Körpers eine Unregelmäßigkeit, an der sich die Phase der Ultraschallwelle ändert, so wird ein Teil der Ultraschallenergie wieder zurückgeworfen. Nach Abgabe eines Ultraschall-Impulses wird das Ultraschallgerät gewöhnlich auf Empfang gestellt, um reflektierte (oder Echo-)Signale vom Körper zurück in elektrische Signale wandeln zu können. Die Zeit, nach der diese Echosignale an dem Empfänger zurückkommen, ist direkt vom Abstand der Reflexionsquelle und von der Schallgeschwindigkeit abhängig. Auch die Stärke des Schallechos ist interessant, weil sie Informationen über die Art einer Störstelle liefert.

Das Echo von Schallwellen kann auf verschiedene Weise dargestellt werden. Einerseits gibt es Geräte mit Verstärker, mit denen die dem empfangenen Ultraschallecho entsprechenden elektrischen Signale verstärkt an den vertikal ablenkenden Platten einer Kathodenstrahlröhre angelegt werden. Der Ausgang eines Zeitgenerators liegt dabei an der horizontalen Ablenkung der Kathodenstrahlröhre. Eine stetige Wiederholung des Impuls/Echo-Vorganges, synchronisiert mit dem Zeitgenerator führt dann zu einem stehenden Bild, sogenannte "A-Ab-tastung", bei der die Zeit der Eindringtiefe proportional ist und vertikale Ablenkungen vorhandene Fehlordnungen signalisieren. Die Intensität dieser vertikalen Ablenkungen ist ein Maß für die Intensität des Echos.

Eine andere übliche Art bildlicher Darstellung von Ultraschallwellen ist die sogenannte B-Abtastung, bei der die Echoinformation dem üblichen Fernsehbild entspricht. d. h. die empfangen Schall-echosignale werden zur Modulierung der Helligkeit des Schirmes je Abtaststelle verwendet. Diese Bildschirmdarstellung wird speziell für Geräte verwendet, bei denen der Ultraschallstrahl im Körper geschwenkt wird, wobei jede Intensitätsinformation eine Abtastlinie des Bildschirmes beansprucht und die aufeinanderfolgenden Positionen hintereinander werden zur Darstellung von aufeinanderfolgenden Linien auf dem Bildschirm verwendet. Mit dieser Technik wird ein Durchlichtbild in einer Ebene abgetastet und das resultierende Bild kann direkt betrachtet werden oder durch eine Photographie oder magnetisches Speicherband gelagert werden.

Der Schallwandler der beschriebenen Geräte hat eine endliche Ausdehnung, wovon die begrenzte Auflösung des aus den Ultraschallwellen gewonnenen Bildes abhängt. Es ist darüberhinaus bekannt, daß Ultraschallwellen mit geeigneten Linsen gerichtet werden können, wie sie im US-Patent Nr. 3,598,559 beschrieben sind und/oder durch Unterteilung des Ultraschallwandlers in verschiedene kleine Teile, die über unterschiedliche Verzögerungsleitungen verbunden sind. Eine Art von Fokussierung kann beispielsweise dadurch erreicht werden, daß schallaufnehmende Bauelemente mit einer Vielzahl von konzentrisch ineinanderliegenden Ringen von Übertragungselementen ausgebildet sind, die über verschiedene Verzögerungsleitungen verbunden sind. Wegen der relativ geringen Schallgeschwindigkeit spielt der Wegunterschied, den die Schallwellen von der Mitte eines Senders zu seiner Randzone beim Hin- und Rückweg über einen theoretischen Brennpunkt hin und zurück überschreiten, eine große Rolle, wenn analog zu elektromagnetischen Wellen eine Strahlbündelung vorgesehen ist. Der Wegunterschied zwischen dem kurzen Weg von Brennpunkt zu Sendermitte zum Weg vom Brennpunkt zum Senderrand muß gemäß eines doppelten Durchlaufes der Ultraschallwellen mit Verzögerungsmitteln ausgeglichen werden. Dabei haben sich elektrische Verzögerungsleitungen auf dem Weg vom elektrischen Erreger zum Schallwandler bewährt.

Es ist ferner bekannt, die Ultraschallmeßtiefe durch Variation der Verzögerungsleitungen zu verändern (dynamische Fokussierung). Nicht veränderbare Verzörungsleitungen gestatten lediglich eine Ultraschallmessung mit einem fest vorgegebenen Brennpunkt, der bei Variation der Verzögerungsleitungen auf unterschiedliche Problemstellungen zur Untersuchung angepaßt werden kann. Schließlich muß noch beachtet werden, daß Ultraschallwellen für tiefergehende Untersuchung länger im Körper verweilen. Bei einer Untersuchung wird die Beobachtung des Körpers in verschiedenen Tiefen verlangt. Bei tiefergehender Untersuchung gleicht sich der Wegunterschied zwischen zentraler Empfangsstelle und Randzonen etwas aus, der durch entsprechende Einstellung der Verzögerungsleitungen zu berücksichtigen ist. Wegen der großen Zahl von Verzögerungsleitungen, die jeweils entsprechend zu steuern sind, ist der technische Umfang und der Gebrauch solcher Geräte sehr kompliziert.

Ein Beispiel einer Einrichtung zur dynamischen Fokussierung bei einem Ultraschall-Darstellungssystem ist in der Publikation "CCD dynamically focussed lenses for ultrasonic systems" aus "CCD 75 proceedings of CCD applications conference naval electronics laboratory center, San Diego, California 1975 (29.—31. Oktober)" zu entnehmen. In dieser Vorpublikation wird vorgeschlagen, einen aus mehreren ringförmigen Elementen bestehenden Ultraschallwandler zu verwenden, wobei jedes Element an einen CCD-Speicher angeschlossen ist. Die Ultraschallinformation wird in diesen Speicher seriell ein- und seriell wieder ausgegeben und auf einem Bildschirm zur Darstellung gebracht. Zur Erreichung einer dynamischen Fokussierung wird die Taktfrequenz jedes einzelnen Speichers während der Eingabe und/oder Ausgabe der Signale in/aus den einzelnen Speichern jeweils dergestalt variiert, daß eine dynamische Fokussierung durch diese Variation erreicht wird. Zu diesem Zweck ist für jeden Speicher ein getrennter Spannungskontrollierter Oszillator und ein Frequenzvervielfacher notwendig. Dies bedingt, wie auch in dieser Publikation ausdrücklich eingeräumt wird, einen sehr großen technischen Aufwand, mit den daraus resultierenden Kosten und einem entsprechenden Raumbedarf.

Der Erfindung liegt die Aufgabe zugrunde, ein Ultraschallechomeßgerät zu schaffen, das eine dynamische Fokussierung ermöglicht und dabei möglichst einfach und folglich wirtschaftlich aufgebaut ist und störungsfrei arbeitet. Eine Aufgabe der Erfindung wird also vor allem darin gesehen, bekannte Geräte erheblich zu verbessern.

Diese Aufgabe wird bei einer Vorrichtung der eingangs bezeichneten Art gelöst durch einen ersten Taktgeber, der jedem der Speicherregister zugeordnet ist, eine Mehrzahl von zweiten Taktgebern, wobei jedem der Speicherregister jeweils ein zweiter Taktgeber mit einer verschiedenen charakteristischen Taktfrequenz zugeordnet ist, eine Zeitsteurschaltung zur Steuerung des Einlesens der Signale von den Elementen in die zugeordneten Speicherregister mit dem Takt der zweiten Taktgeber und des anschließenden Auslesens der gespeicherten Information aus jedem der Speicherregister mit dem Takt des ersten Taktgebers.

Vorteilhafte Ausgestaltungen der Erfindung sind durch die in den Unteransprüchen aufgeführten Merkmale charakterisiert.

Ein Gerät zur Abbildung von Ultraschall, mit dem in einem Körper zur Diagnose ein Ultraschallstrahl ausgesandt wird, welcher teilweise als Echo wieder zurückgeworfen wird, hat häufig einen elektroakustischen Wandler mit einer Vielzahl von Elementen, die in konzentrischen Ringen ineinander in einer Abene angeordnet sind. Vorteilhaft wird ein solcher elektroakustischer Wandler gleichzeitig zur Abgabe der Ultraschallwellen und zum Empfang der reflektierten Ultraschallwellen verwandt. Das erfindungsgemäße Gerät weist ferner eine Vielzahl von Registereinheiten auf, vorzugsweise analog arbeitende Speicherregister des sogenannten "CCD"-Types (Ladungstransportspeicherregister). Jedes Register ist an ein Wandlerelement gekoppelt. An jedes Register ist ein Taktgeber angeschlossen, der Signale mit einer ersten Taktfrequenz erzeugt. Ferner ist eine Vielzahl weiterer Taktgeber vorgesehen, die ebenfalls mit den Analogspeichern verbunden sind. Die zweite Art von Taktgebern liefert Takte mit unterschiedlicher vorgegebener Taktfrequenz. Außerdem sind zeitabhängige Taktgeber vorgesehen, die die Arbeitsweise der ersten und zweiten Taktgeber steuern, so daß wechselweise die Speicher mit der Frequenz der zweiten Taktgeber beladen und anschließend mit der Frequenz der ersten Taktgeber ausgelesen werden. Schließlich ist eine elektrische Verbindung zum Transport der ausgelesenen Signale zu einem Abbildungssystem vorgesehen.

Bei vorliegender Erfindung wird die je Wandlerelement erforderliche Verzögerungsleitung durch eine vorgegebene Frequenz der zweiten Taktgeber ersetzt. Mit der jeweiligen Taktfrequenz wird je eine Zeile entsprechend verzögert in einen Speicher eingelesen. Die solchermaßen mit verzögerter Taktfrequenz eingelesenen Signale können aus dem Speicher mit einer gemeinsamen Frequenz für alle Segmente ausgelesen werden.

Eine andere Ausführungsform der Erfindung wird darin gesehen, daß entgegengesetzt zu dem bisher beschriebenen Einleseverfahren die Speicher mit einer gemeinsamen Frequenz beladen und erst beim Herauslesen mit unterschiedlicher Frequenz die für jedes Segment erforderliche Verzögerung berücksichtigt wird. Außerdem kann beim Ein- und beim Auslesen die Taktfrequenz auf das jeweilige Segment abgestimmt sein.

Weitere vorteilhafte Ausgestaltungen der Erfindungen sind anhand der schematisch dargestellten Zeichnung im folgenden beschrieben. Es zeigen

Fig. 1 ein Gerät der Erfindung im Einsatz,

Fig. 2 im Querschnitt einen Meßkopf des Gerätes mit einem Blockschaltbild der zugehörigen Auswertelektronik,

Fig. 3 einen Teil der elektrischen Schaltung in Blockform, die zur dynamischen Fokussierung vorgesehen ist,

Fig. 4A und B Darstellungen, die hilfreich sind, um die Funktion der Schaltung aus Fig. 3 zu erläutern,

Fig. 5 ein Blockschaltbild einer anderen Schaltung für die Erfindung,

Fig. 6A und B die zugehörigen Darstellungen zur Erklärung der Funktion der Schaltung nach Fig. 5,

Fig. 7 eine weitere Ausführung der erfindungsgemäßen Schaltung in Blockform,

Fig. 8 eine zum Verständis von Fig. 7 geeignete graphische Darstellung.

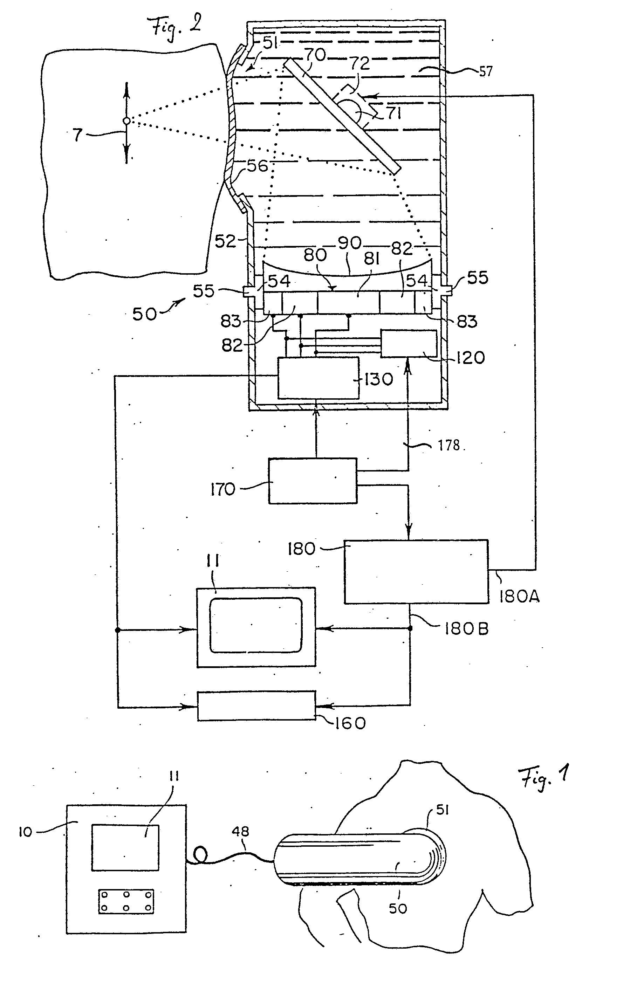

In Fig. 1 werden die äußeren Maße eines Abtastgerätes gemäß der Erfindung im Vergleich mit einem Objekt gezeigt. Das Kontrollpult 10 enthält einen Bildschirm 11, beispielsweise eine Kathodenstrahlröhre, ein einer geeigneten Frontplatte. Außerdem können ein Videobandrecorder oder ein anderer Speicher z.B. auf der Basis photographischer Signale (Bildschirmkopierer), im Kontrollpult 10 enthalten sein, um die Signale zur Anzeige eines Bildes zu liefern. Ferner enthält das Kontrollpult 10 eine Energieversorgung und Teile der Schaltung für die Erzeugung von Zeitsteuersignalen und zum Antrieb des Abtastschallspiegels in dem Meßkopf 50. Der Meßkopf 50 (oder Sonde) ist mit dem Kontrollpult 10 mit einer elektrischen Leitung 48 verbunden. Der Meßkopf 50 des vorliegenden Ausführungsbeispieles ist im wesentlichen zylindrisch/ geformt und hat in der Nähe eines Endes ein Abtastfenster 50, das beispielsweise aus elastisch anchgiebigem Material, wie Silicongummi, besteht. Zur Handhabe des Gerätes wird der Meßkopf 50 in eine vom Bedienenden in der Hand zu haltende Position gebracht, so daß das Abtastfenster 50 auf das abzutastende Objekt elastisch nachgiebigem Material, wie Silicon-Objekt soll beispielsweise der Bereich um das Herz eines Menschen abgetastet werden. Selbstverständlich kann der Meßkopf auch zur Messung anderer Körperstellen oder anderer Objekte verwendet werden, auf die sie mit Handgriff zu richten wäre.

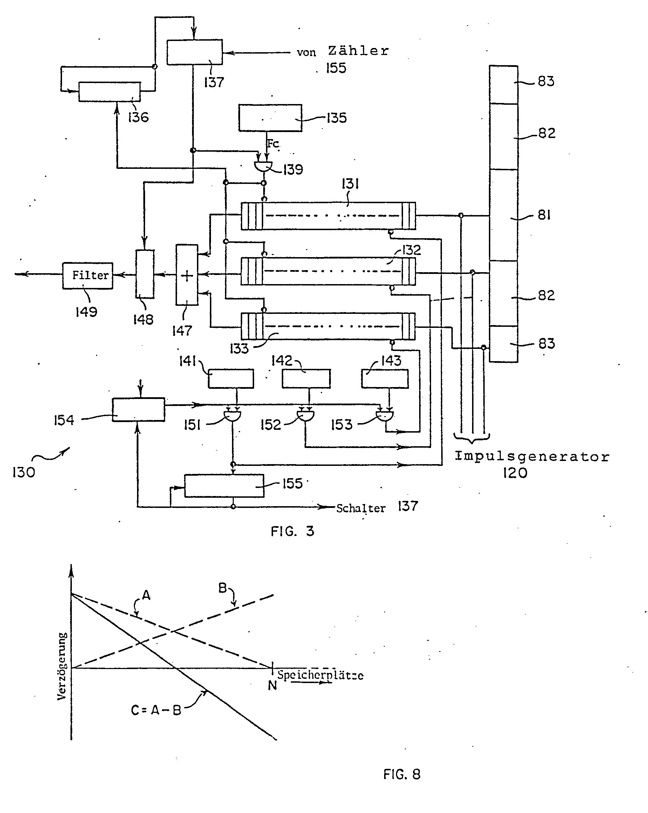

Gemäß Fig. 2 wird der Meßkopf 50 im Querschnitt dargestellt, an den zugehörige Teile der Auswertelektronick angeschlossen sind, die teils in dem Meßkopf 50 und teils im Kontrollpult 10 angeordnet sein können. Das Gehäuse des Meßkopfes 50 schließt eine vordere Schalleitkammer 52, die ein Flüssigkeit enthält, und eine hintere Schallmeßkammer 53, die einen Teil der Elektronik enthält, ein. Beide Kammern 52 und 53 haben Zylinderform mit gleichgroßem Durchmesser, so daß sie mit Hilfe eines Rohres 54, das an seiner Außenseite einen ringförmigen Ansatz 55 besitzt, zu einem Zylinder zusammengesetzt werden können. Das (Innen-)Rohr 54 trägt einen flachen Schallwandler 80 und eine Schallsammellinse 90, von dem die beiden Gehäuseteile voneinander getrennt werden (vgl. US-Patent 3,958,559). Das Abtastfenster 51 befindet sich am Ende der Kammer 52. Rings um die Fensteröffnung ist ein Ansatz vorgesehen, auf dem eine elastisch nachgiebige Membran 56, beispielsweise Silicongummi-Membran, aufgezogen ist. Die vordere Schallkammer 52 ist mit einer Flüssigkeit 57, beispielsweise Wasser, ausgefüllt. Die Membran 56 soll so elastisch sein, daß sie sich mit dem Meßkopf an die Oberfläche des zu messenden Körpers glatt anlegt, um störende Reflektionen von Schallwellen an der Übergangsstelle zwischen der Flüssigkeit des Gerätes und dem Objekt möglichst gering zu halten.

Eine flache, z.B. metallische, schallreflektierende Abtasteinrichtung 70 ist in der Flüssigkeit 57 zwischen der Schalllinse 90 und dem Abtastfenster 51 angeordnet. Natürlich kann die Oberfläche der nicht gewölbt gezeichneten Abtasteinrichtung gebogen sein und selbst reflektierte Schallwellen fokussieren oder zerstreuen. Die Abtasteinrichtung 70, die im folgenden als Abtastschallspiegel oder einfach Abtastspiegel bezeichnet wird, ist an einer senkrecht zur Zeichenebene liegenden Achse 71 befestigt, die durch die Gehäusewand der vorderen Schalleitkammer 52 hindurchgeführt sein kann, um von außen mit einem kleinen elektrischen Motor 72, der die Hinund Herbewegung erzeugt, betrieben zu werden.

Der elektroakustische Wandler 80 ist in eine Vielzahl von Elementen unterteilt, die in konzentrischen Ringen um ein zentrales Element in einer Ebene liegen. In der Darstellung sind nur drei Elemente, bezeichnet mit 81, 82 und 83, anstelle einer unübersichtlichen Vielfalt dargestellt. Selbstverständlich hat der elektroakustische Wandler sehr viel mehr Elemente. Die Elemente des elektroakustischen Wandlers 81 bis 83 sind mit einem elektrischen Impulsgenerator 120 verbunden, von dem sie in bekannter Weise zur Abgabe von Ultraschall angeregt werden können. Die Schallwandlerelemente sind ferner mit der neuen Schaltung zur dynamischen Einstellung des Brennpunktes gemäß der Erfindung verbunden.

Die mit 130 bezeichnete Schaltung arbeitet bevorzugt nur beim Empfang von Schallwellen und leitet elektrische Signale entsprechend den reflektierten Echosignalen des Ultraschalls zwecks Abbildung auf einem Bildschirm weiter. In diesem Schaltkreis können noch Vorverstärkungen und Verstärker vorhanden sein, die im einzelnen in der Figur nicht dargestellt sind. Der Ausgang der Schaltung 130 zur dynamischen Fokussierung des Brennpunktes ist mit einem Bildschirm 11 und einem weiteren Empfänger 16) verbunden, der zur Speicherung des Fernsehbildes durch Videoeinrichtung dient. Eine besonders vorteilhafte Schaltung zur Verstärkerregelung ist in der US-Patentschrift 4,043,181 näher beschrieben. Mit einer solchen Verstärkerregelung werden später eintreffende Echosignale entsprechend ihrer beim Durchgang durch das Körpergewebe erfahrenen Abschwächung verstärkt.

Die Zeittaktschaltung 170 ist vorgesehen, um Pulse mit zeitgleichen Abständen zu erzeugen, mit denen das System synchronisiert wird; die Impulse der Zeittaktschaltung 170 werden dem Impulserzeuger 120 und dem dynamisch fokussierenden Impulsempfänger 130 wechselweise und darüberhinaus dem Abtastspiegelantrieb sowie der Schaltung für die Ablenkung des Elektronenstrahles 180 zugeleitet, so daß taktgleich abwechselnd Ultraschallimpulse abgegeben und empfangen werden und daß die Bewegung des Spiegelantriebes sowie die vertikale und horizontale Ablenkung des Elektronenstrahles der Kathodenstrahlröhre 11 aufeinander abgestimmt werden.

Im großen und ganzen funktioniert die Schaltung wie folgt: Durch ein Triggersignal der Zeittaktschaltung 170, geleitet über die Verbindung 178, wird der Impulsgenerator 120 zur Erzeugung von Impulsen angeregt, die auf die Elemente des elektroakustischen Wandlers 80 übertragen werden, Konzentrische Ringelemente von Ultraschallwandlern werden zur Ausrichtung eines Ultraschallstrahles auf einen Brennpunkt wie bekannt über Verzögerungsleitungen angeregt. Eine weitere Strahlausrichtung ist durch die Linse 90 möglich. Der über den Abtastspiegel 70 in den zu untersuchenden Körper eingebrachte Ultraschallstrahl, dessen, Bereich in der Figur durch gepunktete Linien dargestellt ist, wird nach Shallabgabe durch darauf folgende Umstellung des Gerätes auf Empfang als Echo wieder empfangen. Der elektroakustische Wandler 80 formt nun in umgekehrter Richtung die über den Abtastspiegel zurückgeworfenen Echosignale in elektrische Impulse um.

Die elektrischen Signale werden nach Verarbeitung durch die Schaltung 130 auf einem Bildschirm 11 sichtbar gemacht. Der Bildschirm zeigt einen Schnitt in Richtung der eingesandten Ultraschallwelle durch das Objekt, das sogenannte B-Abtastungsbild. Die zweite Dimension des Bildes ist der Schwenkbereich der Ultraschallwelle, die durch relativ langsames Hin- und Her-bewegen des Abtastspiegels 70 in Richtung des Doppelseitig gerichteten Pfeiles 7 erhalten wird.

Fig. 3 zeigt ein Blockdiagramm der elektrischen Schaltung zur dynamischen Fokussierung für die Schaltung 130, die an die Elemente des Ultraschallwandlers 80 angeschlossen ist. Je Element 81 bis 83 ist ein Speicher 131 bis 133 angeschlossen. Die Speicher sind vorzugsweise Bauelemente, die analog als sogenannte CCD-Bausteine arbeiten. Der Ausgang dieser Speicher ist an einem Additionsglied 147 angelegt, dessen Ausgang am Eingang des Gatters 148 liegt. Der Ausgang des Gatters 148 ist über ein Filter 149, durch das aufgeprägte Taktimpulse ausgefiltert werden, mit dem Bildschirm 11 verbunden.

Taktgeneratoren 141, 142 und 143 sind je einem Schallwandler 81—83 zugeordnet, an dem sie Takte erzeugen, mit denen Information in die Register 131—133 eingespeist wird. Der Taktgenerator 141 erzeugt eine vorgegebene Frequenz $F_0$, der Taktgenerator 142 eine vorgegebene Frequenzy $F_0+\Delta F_1$ und der Taktgenerator 143 eine vorgegebene Frequenz $F_0+\Delta F_2$. Die Ausgänge der Taktgeneratoren 141—143 sind mit den entsprechenden Registereingängen über UND-Gatter 151, 152 und 153 verbunden, durch die das Einlesen in die Speicher gesteuert wird. Der zweite Eingang jedes UND-Gatters 151—153 liegt am Ausgang des Schalters 154. Der Ausgang des UND-Gatters 151 ist außer mit dem Eingang des Speichers 131 mit dem Zähler 155 verbunden, der Impulse mit einer Frequenz $F_0$ zählt.

Im Beispiel der vorliegenden Erfindung hat jedes Register 131—133 n Speicherplätze, demgemäß zählt der Zähler 155 maximal bis zur Zahl n. Wenn der Zähler 155 n Impulse gezählt hat, setzt er sein Zählwerk wieder auf 0 und Schließt den Schalter 154, der von der Zeittaktschaltung 170 eingeschaltet wird. Das in dieser Weise beschriebene Zählsignal kann vorteilhaft dazu verwendet werden, das Gerät von Senden auf Empfang umzustellen. Die Taktfrequenz der Zeittaktschaltung 170 ist ferner so eingestellt, daß nur Echowellen im vorgesehenen Meßbereich der Meßtiefe im Körper empfangen werden.

Ein Impulsgenerator 135 liefert Impulse der Frequenz $F_c$, die mit jedem Speicher 131 bis 133 verbunden sind, und nach denen die Information aus den Speichern ausgelesen wird. Der Ausgang des Generators 135 ist über das Gatter 139 mit den Speichern verbunden. Das Gatter 139 ist auch noch mit einem Zähler 136 verbunden, der maximal m Stellen zählt. Dessen Ausgangssignal setzt beim Erreichen der maximal zulässigen Impulse m den Zähler 136 auf 0 und schaltet den Schalter 137 aus, der auf ein Signal vom Zähler 155 eingeschaltet wird. Das Ausgangssignal des Schalters 137 liegt am UND-Gatter 139 und an dem Gatter 148 an.

Die in Fig. 3 im einzelnen dargestellte Schaltung 130 arbeitet im großen und ganzen wie folgt:

Nach einem Signal der Zeittaktschaltung 170 öffnet sich der Schalter 154, der seinerseits die Gatter 151—153 öffnet. Somit können elektrische Signale von den Elektroakustischen Wandlern 81—83 in den Speichern 131—133 mit der jeweils vorgewählten Taktrate der Taktgeneratoren 141—143 eingelesen werden, bis n Takte, die vom Zähler 155 mitgezählt werden, gespeichert, sind. Der Speicher 131 wird dabei mit der niedrigen Frequenz $F_0$ aufgefüllt, bis der Zähler 155 m Takt gezählt hat, nach denen er sich selbst auf 0 setzt, den Schalter 154 schließt und den Schalter 137 öffnet. Der Schalter 154 schließt auch die Gatter 151—153 und beendet somit den Speichervorgang, dem nun, durch den Schalter 137 ausgelöst, das Auslesen folgt, indem die Gatter 139 und 148 geöffnet werden. Die Information aus den Speichern 131—133 gelangt über Addierer 147 und Gatter 148, sowie Filter 149 auf den Bildschirm 11. Von dem Filter 149 werden aufgeprägten Taktfrequenzen wieder entfernt. Das Auslesen wird mit einem Zähler 136 kontrolliert, der die beim Auszählen aufgeprägten Impulse vom Impulsgenerator 135 bis zu einer Zahl maximal m zählt, wobei im vorliegenden Fall angenommen werden soll, daß n=m ist, wonach der Zähler 136 den Schalter 137 wieder schließt und Gatter 139 und 148 wieder sperrt.

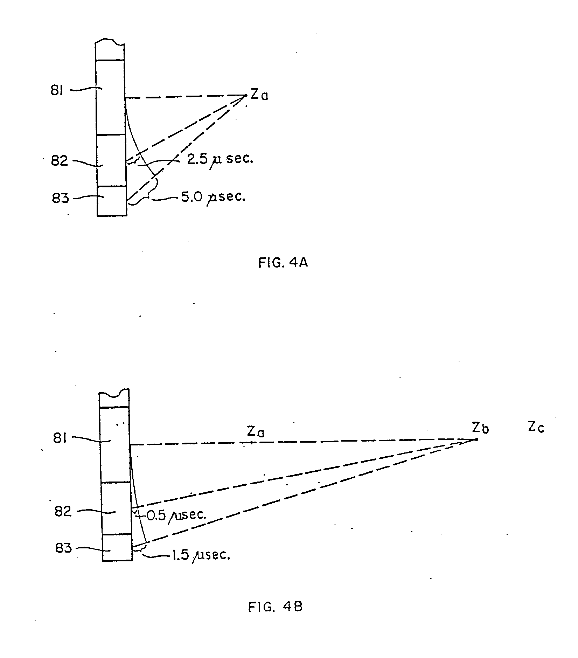

Eine Verzögerung der Ultraschallimpulse wird also erst nach deren Empfang dadurch simuliert, daß die vom Ultraschallwandler gelieferten Signale mit zum Rand des Wandlers steigender, höherer Taktfrequenz eingelesen werden. Dadurch wird der aus den Figuren 4A und B sichtbare Wegunterschied zum Rand ausgeglichen.

Bei der Verwendung der Frequenzen $F_0$, $F_0+\Delta F_1$ und $F_0+\Delta F_2$ ist der Unterschied zum zentralen Wandlerelement n mal $\Delta T_1$ und n mal $\Delta T_2$ (wobei $F=1:T$). Die Verzögerung wird also direkt von den Taktgeneratoren 141—143 eingestellt.

Da der Unterschied der Verzögerung von der Eindringtiefe abhängig ist (vgl. Figuren 4A und B, in denen Messung bei verschiedenen Brennpunkten Z a bis Z c dargestellt ist) ist der Unterschied der Verzögerung der benachbarten Wandlerelemente umgekehrt zur Eindringtiefe einzustellen Einen Teil der Verzögerung übernimmt die Fokussierungslinse 90 (Figur 2), deren Verzögerungswirkung jedoch nicht größer als die Mindestverzögerung bei maximaler Eindringtiefe sein soll. Sollten Meßgeräte mit beliebig großer Eindringtiefe entwickelt werden, dann müßte die Fokussierungslinse 90 entfallen.

In Fig. 4A und 4B sind beispielhaft die Weg- und resultierenden Laufzeitunterschiede für zwei verschiedene Brennpunkte Za und Zb dargestellt. Man kann dieser Figur entnehmen, daß Schallwellen vom vorgesehenen Brennpunkt Za zum ersten Ring 2,5 $\mu$sec und zum zweiten

Empfängerring 5,0 $\mu$sec länger als bis zum Zentrum benötigen. Bei größerer Eindringtiefe ist der Unterschied, den Ultraschallwellen von Brennpunkt zu den äußeren Empfängerzonen benötigen, entsprechend geringer (Fig. 4B).

Wenn man davon ausgeht, daß die Speicher 131—133 mit 1000 Speicherplätzen ausgelegt sind und daß der Zähler 155 bis zur Zahl 1000 zählt, wenn man weiterhin von einer Taktfrequenz $F_0$=20 Megahertz mit einer Verschiebung $\Delta F_1$ von 1,05 Megahertz und $\Delta F_2$=2,22 Megahertz ausgeht, dann werden von den Taktgeneratoren 141—143 Impulse mit einer Frequenz von 20 Megahertz, 21,05 Megahertz und 22,22 Megahertz geliefert.

Bei diesem Beispiel arbeitet das Gerät wie folgt:

Die Zeittaktschaltung 170 gibt das Einschaltsignal etwa zu dem Zeitpunkt, wenn das zuerst eintreffende Signal vom Punkt Za in Segment 81 aufgenommen wird. Die taktweise Speicherung der Signale im Speicher 131 dauert bei der Frequenz von 20 Megahertz 0,05 $\mu$sec pro Takt des Taktgebers 141. Der gesamte Eintaktvorgang bei 1000 Speicherplätzen dauert dann also 50 $\mu$sec. Der Zähler 155 zählt diese 1000 Takte genau mit, bis der Speicher vollständig aufgefüllt ist, so daß auf dem zuletzt aufgefüllten Speicherplatz ein Echoinformation vom Brennpunkt Za vorhanden ist. (Die in dem dargestellten bevorzugten Ausführungsbeispiel zur Anwendung kommenden Ladungstransportspeicher arbeiten dergestalt, daß die am einen Ende eingegebene Information während des Speichervorganges durchläuft, in der Darstellung der Fig. 3 beispielsweise von rechts nach links, so daß die am Eingang des Speichers zuerst eingegebene Information nach dem Speichervorgang dem Ausgang des Speichers am nächsten abgespeichert ist und die folgenden Informationen sich daran, in der Darstellung der Figur von links nach rechts anschließen). Der Speichervorgang in den Speichern 132 und 133 wird nun von den Taktgebern 142 bzw. 143 mit den höheren Frequenzen 21,05 Megahertz und 22,22 Megahertz getaktet, so daß das Einspeichern pro Speicherplatz 0,0475 bzw. 0,045 $\mu$sec dauert. Das Einlesen der Signale auf die 1000 Speicherplätze dauert demzufolge 47,5 $\mu$sec bzw. 45 $\mu$sec.

Der Einlesevorgang wird, wie erwähnt, durch den Schalter 154 beendet, wenn der Zähler 155 tausend Impulse des Taktgebers 141 gezählt hat, im Beispiel also nach 50 $\mu$sec. Demzufolge wird in die Speicher 132 und 133 zeitlich länger eingelesen, als zur Besetzung der Speicherplätze nötig ist. Da der Speichervorgang zum gleichen Zeitpunkt wie beim Speicher 131 beendet wird, wird die zusätzliche Information aus der Anfangsphase des Speichervorganges nach Besetzung des ausgangsseitigen (in der Figur am weitesten links liegenden) Speicherplatzes gelöscht. Eine einfache Überlegung zeigt, daß nach Beendigung des Speichervorganges auch bei den Speichern 132 und 133 genau die vom Brennpunkt Za kommende Information auf dem dem Speicherausgang nächstliegenden Speicherplatz abgespeichert ist. Die gelöschte Information entspricht genau dem Unterschied der Laufzeit der Schallwelle vom Brennpunkte Za zu den äußeren Ringen 82 bzw. 83 des Schallwandlers 80.

In Fig. 4B ist ein vom Schallwandler 80 weiter entfernter Brennpunkt Zb dargestellt, dessen Entfernung von Za einer Laufzeit von 40 $\mu$sec entspricht. Das von diesem Brennpunkt auf das Wandlerelement 81 auftreffende Signal wird demzufolge 40 $\mu$sec nach Beginn des Einlesevorganges im Speicher 131 abgespeichert, und zwar, vom Speicherausgang gezählt, auf dem 800. Speicherplatz, also 200 Plätze vom Speichereingang. Wie eine einfache Rechnung zeigt, enthält der entsprechende Speicherplatz in den Speichern 132 und 133 aufgrund der erhöhten Taktfrequenzen beim Einlesen Signale, welche um 0,5 $\mu$sec (200×0,0025) bzw. 1,0 $\mu$sec (200×0,005 $\mu$sec) später an den zugeordneten Wandlerringen 82 bzw. 83 eingetroffen sind. Dieser Unterschied entspricht, wie Fig. 4B zeigt, genau dem Laufzeitunterschied der Schallwellen vom Brennpunkt Zb zu den Ringen 82 bzw. 83. Die gleiche Überlegung läßt sich für jeden Brennpunkt anstellen, der zwischen Za und Zc liegt, wobei Zc den Brennpunkt markiert, auf den der Ultraschallstrahl aufgrund der Linse 90 ohne zusätzliche elektronische Verzögerung fokussiert ist.

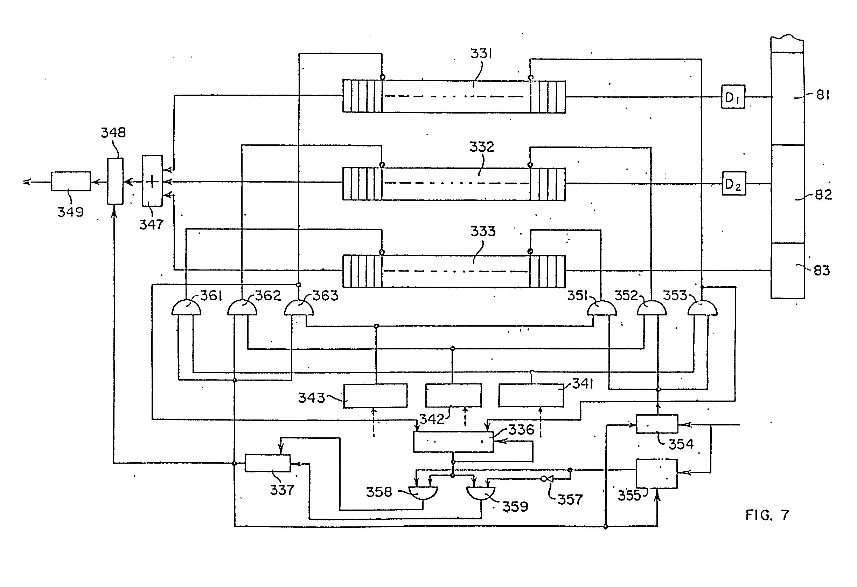

Die durch die verschiedene Eintaktung vorgegebene elektrische Verschiebung des Brennpunktes wirkt sich natürlich erst dann aus, wenn die Speicher mit gleicher Frequenz wieder ausgelesen werden. Da der zuerst ausgelesene, dem Speicherausgang am nächsten liegende Speicherplatz, wie obige Überlegungen zeigen, in sämtlichen Speichern 131 bis 133 das dem Brennpunkt Za entsprechende Signal und der letzte Speicherplatz das dem Brennpunkt Zc entsprechende Signal enthält, kommen die Signale auch gleichzeitig zu dem Additionsglied 147 und dem-zufolge zum Bildschirm 11. Das heißt, das Bild ist für den gesamten Bereich zwischen den Brennpunkten Za und Zc dynamisch fokussiert. Eine noch stärkere Verschiebung kann erreicht werden, wenn mit sich in umgekehrter Reihenfolge ändernden Taktfrequenzen ausgelesen wird. Das ist schaltungstechnisch relativ einfach, weil eine dazu erforderliche Steuerung bereits zur Aufnahme und Speicherung der Signale vorgesehen ist. Eine entsprechende Schaltung ist in Fig. 7 dargestellt und wird weiter unten beschrieben.

Eine besonders vorteilhafte Einrichtung der Erfindung wird darin gesehen, daß Bauelemente zur Einstellung der Taktzahl jedes Registers von einer gemeinsamen Antriebswelle betätigbar so verbunden sind, daß sie die Taktzahl proportional zum Abstand des Brennpunktes und in Relation zur Änderung der Taktzahl der benachbarten Speicher gleichzeitig festlegen.

Es sollte in diesem Zusammenhang darauf hingewiesen werden, daß die Frequenzen der Taktgeber 141 bis 143 verschieden, aber jede für sich bei der normalen Ausführungsform der Erfindung konstant sind. Eine derartige Ausführungsform ist in aller Regel völlig ausreichend, um eine hinreichend scharfe dynamische Fokussierung zu erhalten. Lediglich unter bestimmten Umständen ist die zusätzliche gemeinsame geringfügige Änderung der Taktfrequenzen sinnvoll, um eine noch bessere Korrektur auch kleinster Fehlfokussierungen zu ermöglichen.

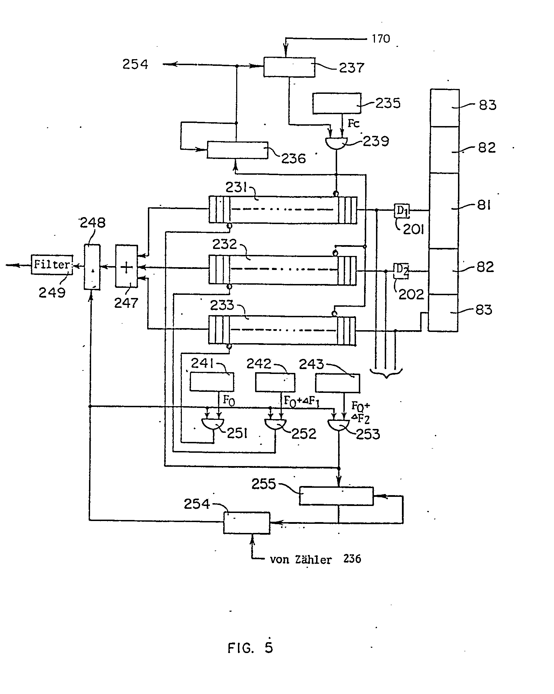

In weiterer Ausgestaltung der Erfindung ist in Fig. 5 eine elektrische Schaltung mit entsprechenden Blocksymbolen dargestellt, bei denen die entsprechenden Bauteile mit Bezugszeichen versehen sind, die sich von den Bezugszeichen der Fig. 3 durch eine 2 in der Hunderterstelle unterscheiden, wenn sie entsprechende Aufgaben erfüllen.

Die in Fig. 5 im einzelnen dargestellte Schaltung 130 arbeitet im großen und ganzen wie folgt:

Nach einem Signal des Zeitgenerators 170 öffnet sich der Schalter 237, der seinerseits das Gatter 239 öffnet. Somit können elektrische Signale von den elektroakustischen Wandlern 81 bis 83 in die Speicher 231 bis 233 mit der Taktrate Fc des Taktgenerators 235 eingelesen werden, bis n Takte, die vom Zähler 236 mitgezählt werden, gespeichert sind. Die Speicher 231 bis 233 werden dabei mit der Frequenz Fc aufgefüllt, bis der Zähler 236 n Takte gezählt hat, nach denen er sich selbst auf 0 setzt, den Schalter 237 schließt und den Schalter 254 öffnet. Der Schalter 237 schließt auch das Gatter 239 und beendet somit den Speichervorgang, dem nun, durch den Schalter 254 ausgelöst, das Auslesen folgt, indem die Gatter 251 bis 253 und 248 geöffnet werden. Die Information aus den Speichern 231 bis 233 gelangt über Addierer 247 und Gatter 248, sowie Filter 249 auf den Bildschirm 11. Von dem Filter 249 werden aufgeprägte Taktfrequenzen wieder entfernt. Das Auslesen wird mit einem Zähler 255 kontrolliert, der die beim Auszählen aufgeprägten Impulse vom Impulsgenerator 235 bis zu einer Zahl maximal m zählt, wobei m gleich n ist, wonach der Zähler 255 den Schalter 254 und die Gatter 239 und 248 wieder schließt.

Die elektrische Schaltung gemäß Fig. 5 unterscheidet sich also von der in Fig. 3 beschriebenen dadurch, daß die Taktgeber 241 bis 243 mit ihren verschiedenen Frequenzen, die, wie in der Figur dargestellt, von $F_0$ für den Taktgeber 241 über $F_0+\Delta F_1$ für den Taktgeber 242 auf $F_0+\Delta F_2$ für den Taktgeber 243 ansteigen, zum Auslesen statt zum Einlesen der Information verwendet werden. Der Taktgeber 241 ist mit dem Speicher 233 verbunden, welcher dem äußersten Ring 83 des Schallwandlers zugeordnet ist, d.h. das Auslesen erfolgt bei dieser Ausführungsform mit von dem äußersten Schallwandlerring 83 zu dem innersten Schallwandlerring 81 ansteigenden Frequenzen. Das Einlesen erfolgt bei dieser Ausführungsform dagegen mit Hilfe eines mit allen Speichern 231 bis 233 verbundenen Taktgebers 235, d.h. mit der gleichen Frequenz für alle Speicher.

Zusätzlich sind Festverzögerungsglieder 201 und 202 in die Einleseleitungen der weiter innen liegenden Speicher 231 und 232 geschaltet. Diese verzögern das Einlesen der Signale von den Weiter innen liegenden Schallwandlerringen 82, 81, um von außen nach innen zunehmende feste Beträge.

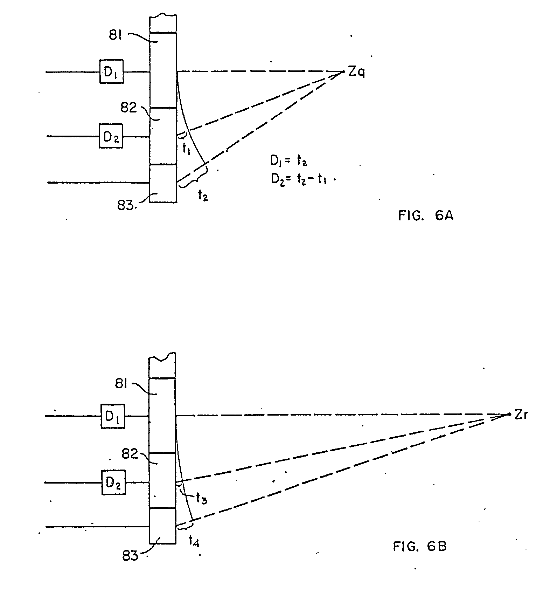

Die Arbeitsweise einer Schaltung gemäß Fig. 5 ist anhand der Fig. 6A und 6B näher erläutert. Fig. 6A zeigt die Abbildungsverhältnisse bei einem relativ naheliegenden Brennpunkt Zq, Fig. 6B bei einem weiter entfernten Brennpunkt Zr. Wie in Fig. 6A dargestellt ist, entspricht die Verzögerung $D_1$ beim mittleren Wandlerelement 81 der Laufzeitdiffernz $t_2$ des Ultraschalls vom Brennpunkt Zq zum mittleren Wandlerelement 81 gegenüber der Laufzeit vom Brennpunkt Zq zum äußersten Wandlerelement 83. Die Festverzögerung $D_1$ in der Einleseleitung des Wandlerelementes 82 entspricht der Differnz der entsprechenden Laufzeitunterschiede zwischen den den Elementen 83 und 82 zugeordneten Laufzeiten $t_2$ und $t_1$.

Aufgrund dieser festen Verzögerungen ist ein Brennpunkt Zq fest eingestellt, der nun durch das erfindungsgemäß mit verschiedenen Frequenzen erfolgende Auslesen der Signale aus den Speichern 231 bis 233 für verschiedene weiter entfernte Fokuspunkte dynamisch angepaßt wird. Die Vorgänge sind dabei prinzipiell die gleichen wie zuvor beschrieben wobei der Unterschied im wesentlichen darin besteht, daß durch das mit verschiedenen Frequenzen erfolgende Auslesen die Signale des innersten Wandlerelementes umsomehr beschleunigt werden, je weiter der Brennpunkt entfernt liegt. Dadurch wird die Festverzögerung $D_1$ jeweils im notwendigen Umfang kompensiert, so daß auch hier eine dynamische Fokussierung erreicht wird.

In Fig. 7 ist eine Ausführungsform dargestellt, bei der die Taktfrequenz der Speicher 331 bis 333 sowohl beim Einlesen, als auch beim Auslesen progressiv verschieden ist, wobei beim Einlesen die höchste Frequenz $F_0+\Delta F_1$ des Taktgebers 343 dem äußersten Wandlerelement 83 mit dem zugehörigen Speicher 333 zugeordnet ist, während die niedrigste Taktfrequenz $F_0-\Delta F_1$ des Taktgebers 341 beim Einlesen dem zentralen Wandlerelement 81 zugeordnet ist. Dies ist aus der Figur deutlich zu erkennen. Der Taktgeber 342 hat die entsprechende mittlere Frequenz $F_0$.

Beim Auslesen ist die Zuordnung umgekehrt, d.h. die höchste Frequenz des Taktgebers 343 steuert das Auslesen aus dem dem zentralen Wandlerelement 81 zugeordneten Speicher 331. Durch die dargestellte Schaltung mit den Gattern 351 bis 353 und 361 bis 363 wird in Verbindung mit dem Schalter 354 das Einlesen und mit dem Schalter 337, das Auslesen gesteuert.

Das Flip-Flop 355 steuert den Umschaltvorgang mit Hilfe des Zählers 336, der beim Einlesen die Signale des Taktgebers 341 und beim Auslesen die des Taktgebers 343 zählt. Die Gatter 358 und 359 dienen zusammen mit dem Inverter 357 in leicht erkennbarer Weise ebenfalls der Steuerung des Einlese- und Auslesevorganges. Wenn durch ein Signal von der Zeittaktschaltung 170, wie bei den anderen Ausführungsformen, der Schalter 354 eingeschaltet wird, wird gleichzeitig das Flip-Flop 355 auf logisch 1 gesetzt und somit das UND-Gatter 358 geöffnet. Wen der Zähler 336 beim Einlesen seinen höchsten Zählerstand N erreicht hat, gibt er ein Ausgangssignal und setzt sich selbst zurück. Da das UND-Gatter 358 geöffnet ist, gelangt das Zählerausgangssignal an den Schalter 337, wodurch der Auslesevorgang eingeschaltet wird, indem die UND-Gatter 361 bis 363 und das Gatter 348 geöffnet werden. Gleichzeitig wird der Einleseschalter 354 geschlossen und das Flip-Fop 355 zurückgesetzt. Damit steht am Ausgang des Flip-Flops 355 eine logische 0 an, so daß über den Inverter 357 das UND-Gatter 359 geöffnet wird. Wenn nun beim Auslesen der Zähler 336 beim Zählen der Signale des Taktgebers 343 wieder den Zählerstand N erreicht hat, erzeugt er wiederum ein Ausgangssignal, welches über des geöffnete UND-Gatter 359 den Schalter 337 ausschaltet und somit das Auslesen beendet.

Bei der in Fig. 7 dargestellten Ausführungsform sind Festverzögerungsglieder mit den Verzögerungen $D_1$ und $D_2$ vorgesehen welche für sich allein genommen eine Fokussierung auf einen mittleren Brennpunkt bewirken.

Bei der Ausführungsform nach Fig. 7 werden beide im Zusammenhang mit den Fig. 3 und 5 beschriebenen Formen der Verzögerung auf entgegengesetzten Seiten des mittleren Brennpunktesangewendet. Beim Einlesen werden die Signale von mehr der Mitte zu gelegenen Schallwandlerelementen zunehmend mehr verzögert, weil sie mit einer zunehmend geringeren Frequenz eingelesen werden, so daß beispielsweise die Information, die in der letzten Stufe des zugeordneten Speichers zur Ruhe kommt, von der Einrichtung zur dynamischen Fokussierung maximal verzögert worden ist. Die relative Verzögerung aufgrund des Einlesevorganges nimmt für die früher liegenden Stufen (ähnlich der Situation bei der Ausführungsform nach Fig. 3) zunehmend ab, wie dies durch die gestrichelte Linie "A" in der Fig. 8 dargestellt ist. Für den Auslesevorgang gilt die umgekehrte Situation (ähnlich der Ausführungsform nach Fig. 5). Dies ist in Fig. 8 durch die gestrichelte Linie "B" dargestellt. Die Gesamtverzögerung durch das System zur dynamischen Fokussierung ist in Fig. 8 durch die Linie "C" (gleich A—B) dargestellt. Etwa in der Mitte der Speicher (die etwa der Mitte des Tiefenbereiches der Ultraschalleinrichtung entspricht) ist die Verzögerung 0 und nur die erwähnten Festferzögerungseinrichtungen sind wirksam.

**Ansprüche**

1. Vorrichtung zur bildlichen Darstellung eines Körpers mit

— einer Sendeeinrichtung (130, 80) zum Ausstrahlen von Energie in den Körper,
— einem Wandler (80) zur Umwandlung von aus dem Körper reflektierter Energie in elektrische Signale, der in eine Mehrzahl getrennter Elemente (81—83) unterteilt ist,
— einer Mehrzahl von Speicherregistern (131—133), wobei der Eingang jedes Speicherregisters (131—133) mit einem zugeordneten Wandlerelement (81—83) verbunden ist und,
— einer Summierungsschaltung (147) zum Zusammenfassen der aus den Speicherregistern (131—133) ausgelesenen Information, um ein Bildsignal zu erhalten, gekennzeichnet durch,
— einen ersten Taktgeber (135), der jedem der Speicherregister (131—133) zugeordnet ist,
— eine Mehrzahl von zweiten Taktgebern (141—143), wobei jedem der Speicherregister (131—133) jeweils ein zweiter Taktgeber (141—143) mit einer Verschiedenen charakteristischen Taktfrequenz zugeordnet ist,
— eine Zeitsteuerschaltung (170, 136, 137, 154, 155) zur Steuerung des Einlesens der Signale von den Elementen (81—83) in die zugeordneten Speicherregister (131—133) mit dem Takt der zweiten Taktgeber (141—143) und des anschließenden Auslesens der gespeicherten Information aus jedem der Speicherregister (131—133) mit dem Takt des ersten Taktgebers (135).

2. Vorrichtung zur bildlichen Darstellung eines Körpers mit

— einer Sendeeinrichtung (130, 80) zum Ausstrahlen von Energie in den Körper,
— einem Wandler (80) zur Umwandlung von aus dem Körper reflektierter Energie in elektrische Signale, der in eine Mehrzahl getrennter Elemente (81—83) unterteilt ist,
— einer Mehrzahl von Speicherregistern (231—233), wobei der Eingang jedes Speicherregisters (231—233) mit einem zugeordneten Wandlerelement (81—83) verbunden ist und,
— einer Summierungsschaltung (247) zum Zusammenfassen der aus den Speicherregistern (231—233) ausgelesenen Information, um ein Bildsignal zu erhalten gekennzeichnet durch,
— einen ersten Taktgeber (235), der jedem der Speicherregister (231—233) zugeordnet ist,
— eine Mehrzahl von zweiten Taktgebern (241—243), wobei jedem der Speicherregister (231—233) jeweils ein zweiter Taktgeber (241—243) mit einer verschiedenen charakteristischen Taktfrequenz zugeordnet ist,
— eine Zeitsteuerschaltung (170, 236, 237, 254, 255) zur Steuerung des Einlesens der Signale von den Elementen (81—83) in die zugeordneten Speicherregister (231—233) mit dem Takt des ersten Taktgebers (235) und des anschließenden Auslesens der gespeicherten Information aus jedem der Speicherregister (231—233) mit dem Takt der zweiten Taktgeber (241—243).

3. Vorrichtung zur bildlichen Darstellung eines Körpers mit

— einer Sendeeinrichtung (130—80) zum Ausstrahlen von Energie in den Körper,
— einem Wandler (80) zur Umwandlung von aus dem Körper reflektierter Energie in elektrische Signale, der in eine Mehrzahl getrennter Elemente (81—83) unterteilt ist,
— einer Mehrzahl von Speicherregistern (331—333) wobei der Eingang jedes Speicherregisters (331—333) mit einem zugeordneten Wandlerelement (81—83) verbunden ist und,
— einer Summierungsschaltung (347) zum Zusammenfassen der aus den Speicherregistern (331—333) ausgelesenen Signale, um ein Bildsignal zu erhalten gekennzeichnet durch eine Mehrzahl von Taktgebern (341—343) mit verschiedenen charakteristischen Taktfrequenzen,
— eine Zeitsteuereinrichtung (170, 336, 337, 351—355, 357—359, 361—363) zur Steuerung des Einlesens der Signale von den Elementen (81—83) in die zugeordneten Speicherregister (331—333) mit verschiedenen Taktfrequenzen, die von den verschiedenen Taktgebern (341—343) erzeugt werden, wobei eine erste Zuordnung zwischen den Taktgebern (341—343) und den Speicherregister (331—333) besteht und des anschließenden Auslesens der gespeicherten Information aus den Speicherregistern (331—333) mit verschiedenen Taktfrequenzen, die von den verschiedenen Taktgebern (341—343) erzeugt werden, wobei eine von der ersten verschiedene zweite Zuordnung zwischen den Taktgebern (341—343) und den Speicherregistern (331—333) besteht.

4. Vorrichtung nach einem der Ansprüche 1—3, dadurch gekennzeichnet, daß Ultraschallenergie verwendet wird.

5. Vorrichtung nach einem der Ansprüche 1—4, dadurch gekennzeichnet, daß der Energiewandler (80) aus einem zentralen Element (81) und mehreren dieses umgebenden ringförmingen Elementen (82, 83) besteht.

6. Vorrichtung nach Anspruch 5 dadurch gekennzeichnet, daß derjenige von den Taktgebern (141—143; 341—343), der beim Einlesen dem zentralen Element (81) zugeordnet ist, eine niedrigere charakteristische Taktfrequenz hat, als die den ringförmigen Elementen (82, 83) zugeordneten Taktgeber (142, 143; 342, 343).

7. Vorrichtung nach Anspruch 6, dadurch gekennzeichnet, daß die den ringförmigen Wandlerelementen (82, 83) zugeordneten Taktgeber (242, 243; 342, 343) eine zunehmend höhere charakteristische Taktfrequenz haben.

8. Vorrichtung nach Anspruch 5 dadurch gekennzeichnet, daß derjenige von den Taktgebern (241—243; 341—343), der beim Auslesen dem zentralen Element (81) zugeordnet ist, eine höhere Taktfrequenz hat als die den ringförmigen Elementen (82, 83) zugeordneten Taktgeber (242, 243; 342, 341).

9. Vorrichtung nach Anspruch 8, dadurch gekennzeichnet, daß die den ringförmigen Wandlerelementen (82, 83) zugeordneten Taktgeber (242, 243; 342, 341) eine zunehmend niedrigere charakteristische Taktfrequenz haben.

10. Vorrichtung nach einem der Ansprüche 1—9, dadurch gekennzeichnet, daß die Speicherregister (131—133; 231—233; 331—333) analoge Ladungstransportspeicher (CCD-Speicher) sind.

## Claims

1. Apparatus for the pictorial representation of a body with

— a transmitter device (130, 80) for the emission of energy into the body,
— a transducer (80), for the conversion of energy reflected from the body into electrical signals, which is subdivided into a plurality of separate elements (81—83),
— a plurality of memory registers (131—133), the input of each memory register (131—133) being connected with an associated transducer element (81—83) and,
— an integrator hookup (147) for the compilation of the information read off from the memory registers (131—133) in order to obtain a pictorial signal, characterised by,
— a first clock generator (135) which is associated with each of the memory registers (131—133),
— a plurality of second clock generators (141—143), each of the memory registers (131—133) being associated with a second clock generator (141—143) with a different characteristic pulse frequency,

— a time-control circuit (170, 136, 137, 154, 155) for controlling the input of the signals from the elements (81—83) into the associated memory registers (131—133) with the pulse of the second clock generators (141—143) and of the subsequent read out of the stored information from each of the memory registers (131—133) with the pulse of the first clock generator (135).

2. Apparatus for the pictorial representation of a body with

— a transmitter device (130, 80) for the emission of energy into the body,
— a transducer (80) for the conversion of energy reflected from the body into electrical signals, which is subdivided into a plurality of separate elements (81—83),
— a plurality of memory registers (231—233), the input of each memory register (231—233) being connected with an associated transducer element (81—83) and,
— an integrator hookup (247) for the compilation of the information read off from the memory registers (231—233) in order to obtain a pictorial signal, characterised by
— a first clock generator (235) which is associated with each of the memory registers (231—233),
— a plurality of second clock generators (241—243), each of the memory registers (231—133) being associated with a second clock generator (241—243) with a different characteristic pulse frequency,
— a time control circuit (170, 236, 237, 254, 255) for controlling the input of the signals from the elements (81—83) into the associated memory registers (231—233) with the pulse of the first clock generator (235) and of the subsequent read out of the stored information from each of the memory registers (231—233) with the pulse of the second clock generators (241—243).

3. Apparatus for the pictorial representation of a body with

— a transmitter device (130, 80) for the emission of energy into the body,
— a transducer (80), for the conversion of energy reflected from the body into electrical signals, which is subdivided into a plurality of separate elements (81—83),
— a plurality of memory registers (331—33), the input of each memory register (331—33) being connected with an associated transducer element (81—83) and,
— an integrator hookup (347) for the compilation of the signals read off from the memory registers (331—33) in order to obtain a pictorial signal, characterised by,
— a plurality of clock generators (341—343) with different characteristic pulse frequencies,

— a time-control device (170, 336, 337, 351— 355, 357—359, 361—363) for controlling the input of the signals from the elements (81—83) into the associated memory registers (331—333) with different pulse frequencies, which are produced by the different clock generators (341—343), whereby a first correlation exists between the clock generators (341—343) and the memory registers (331—333) and of the subsequent read out of the stored information from the memory registers (331—333) with different pulse frequencies, which are produced by the different clock generators, whereby there exists a secondary correlation, different from the first, between the clock generators (341—343) and the memory registers (331—333).

4. Apparatus according to one of claims 1— 3, characterised in that ultrasonic energy is used.

5. Apparatus according to one of claims 1— 4, characterised in that the energy transducer (8) consists of a central element (81) and several ring-shaped elements (82, 83) surrounding this.

6. Apparatus according to claim 5, characterised in that one of the clock generators (141—143; 341—343) which, in the case of the input is associated with the central element (81) has a lower characteristic pulse frequency than the clock generators (142, 143; 342, 343) associated with the ring-shaped elements (82, 83).

7. Apparatus according to claim 6, characterised in that the clock generators (242, 243; 342, 343) associated with the ring-shaped transducer elements (82, 83) have an increasingly higher characteristic pulse frequency.

8. Apparatus according to claim 5, characterised in that one of the clock generators (241—243; 341—343) which, in the case of the read out is associated with the central element (81) has a higher pulse frequency than the clock generators (242, 243; 342, 341) associated with the ring-shaped elements (82, 83).

9. Apparatus according to claim 8, characterised in that the clock generators (242, 243; 342, 341) associated with the ring-shaped transducer elements (82, 83) have an increasingly lower characteristic pulse frequency.

10. Apparatus according to one of claims 1—9, characterised in that the memory registers (131—133; 231—233; 331—333) are analogue registers of the charge transfer type (CCD memories).

**Revendications de Brevet**

1. Appareil pour la représentation en images d'un corps comprenant:

— un dispositif émetteur (130, 80) pur le rayonnement d'énergie dans le corps;

— un convertisseur (80) pour la conversion de l'énergie réfléchie par l'intérieur du corps en signaux électriques, sous divisés en une pluralité d'éléments séparés (81 à 83);

— une pluralité de registres d'emmagasinage (131 à 133), l'entrée de chaque registre (131 à 133) étant reliée à un élément convertisseur coordonné (81 à 83) et;

— un circuit sommateur (147) pour faire la synthèse des informations lues dans les registres d'emmagasinage (131 à 133) afin d'obtenir un signal d'image, caractérisé en ce qu'il comprend:

— un premier générateur d'horloge (135) coordonné à chacun des registres d'emmagasinage (131 à 133);

— une pluralité de seconds générateurs d'horloge (141 à 143), chacun des registres d'emmagasinage (131 à 133) étant chaque fois coordonné à un second générateur d'horloge (141 à 143) ayant une fréquence d'horloge caractéristique différente;

— un circuit de temporisation (170, 136, 137, 154, 155) pour la commande de l'introduction des signaux provenant des éléments (81 à 83) dans les registres d'emmagasinage coordonnés (131 à 133) avec la fréquence des seconds générateurs d'horloge (141 à 143) et pour la lecture consécutive de l'information emmagasinée à partir de chaque registre d'emmagasinage (131 à 133) avec la fréquence du premier générateur d'horloge (135).

2. Appareil pour la représentation en images d'un corps comprenant:

— un dispositif émetteur (130, 80) pour la rayonnement d'énergie dans le corps;

— un convertisseur (80) pour la conversion de l'énergie réfléchie par l'intérieur du corps en signaux électriques, sous divisés en une pluralité d'éléments séparés (81 à 83);

— une pluralité de registres d'emmagasinage (231 à 233), l'entrée de chaque registre (231 à 233) étant reliée à un élément convertisseur coordonné (81 à 83) et;

— un circuit sommateur (247) pour faire la synthèse des informations lues dans les registres d'emmagasinage (231 à 233) afin d'obtenir un signal d'image, caractérisé en ce qu'il comprend:

— un premier générateur d'horloge (235) coordonné à chacun des registres d'emmagasinage (231 à 233);

— une pluralité de seconds générateurs d'horloge (241 à 243), chacun des registres d'emmagasinage (231 à 233) étant chaque fois coordonné à un second générateur d'horloge (241 à 243) ayant une fréquence d'horloge caractéristique différente;

— un circuit de temporisation (170, 236, 237, 254, 255) pour la commande de l'introduction des signaux provenant des éléments (81 à 83) dans les registres d'emmagasinage coordonnés (231 à 233) avec la fréquence du premier générateur d'horloge (235) et pour la lecture consécutive de l'information emmagasinée à partir de chaque registre d'emmagasinage (231 à 233) avec la fréquence des seconds générateurs d'horloge (241 à 243).

3. Appareil pour la représentation en images d'un corps comprenant:

— un dispositif émetteur (130, 80) pour le rayonnement d'énergie dans le corps;
— un convertisseur (80) pour la conversion de l'énergie réfléchie par l'intérieur du corps en signaux électriques, sous divisés en une pluralité d'éléments séparés (81 à 83);
— une pluralité de registres d'emmagasinage (331 à 333), l'entrée de chaque registre (331 à 333) étant reliée à un élément convertisseur coordonné (81 à 83) et;
— un circuit sommateur (347) pour faire la synthèse des informations lues dans les registres d'emmagasinage (331 à 333) afin d'obtenir un signal d'image, caractérisé en ce qu'il comprend:
— une pluralité de générateurs d'horloge (341 à 343) ayant des fréquences d'horloge caractéristiques différentes;
— un circuit de temporisation (170, 336, 337, 351 à 355, 357 à 359, 361 à 363) pour la commande de l'introduction des signaux provenant des éléments (81 à 83) dans les registres d'emmagasinage coordonnés (331 à 333) avec des fréquences d'horloge différentes, créés par les différents générateurs d'horloge (341 à 343) une première coordination existant entre les générateurs d'horloge (341 à 343) et les registres d'emmagasinage (331 à 333) et pour la lecture consécutive de l'information emmagasinée dans les registres d'emmagasinage (331 à 333) avec des fréquences d'horloge différentes, créées par les différents générateurs de fréquence (341 à 343), une deuxième coordination différente de la première existant entre les générateurs de fréquence (341 à 343) et les registres d'emmagasinage (331 à 333).

4. Appareil selon l'une des revendications 1 à 3 caractérisé en ce que l'on utilise de l'énergie ultrasonique.

5. Appareil selon l'une des revendications 1 à 4 caractérisé en ce que le convertisseur d'énergie (80) comprend un élément central (81) et plusieurs éléments circulaires (82, 83) l'entourant.

6. Appareil selon la revendication 5, caractérisé en ce qu celui des générateurs de fréquence (141 à 143, 341 à 343) qui, pendant l'introduction est coordonné à l'élément central (81), a une fréquence d'horloge caractéristique inférieure à celle des générateurs d'horloge (142, 143, 342, 343) coordonnés aux éléments circulaires (82, 83).

7. Appareil selon la revendication 6, caractérisé en ce que les générateurs de fréquence (242, 243; 342, 343) coordonnés aux éléments convertisseurs circulaires (82, 83) ont une fréquence d'horloge caractéristique croissante.

8. Appareil selon la revendication 5 caractérisé en ce que celui des générateurs de fréquence (241 à 243, 341 à 343) qui, pendant la lecture, est coordonné à l'élément central (81), a une fréquence d'horloge plus élevée que les générateurs de fréquence (242, 243; 342, 341) coordonnés aux éléments circulaires (82, 83).

9. Appareil selon la revendication 8, caractérisé en ce que les générateurs de fréquence (242, 243; 342, 341) coordonnés aux éléments convertisseurs circulaires (82, 83) ont des fréquences d'horloge caractéristiques décroissantes.

10. Appareil selon l'une des revendications 1 à 9, caractérisé en ce que les registres d'emmagasinage (131 à 133; 331 à 333) sont des mémoires à transport de charge analogiques (mémoires CCD).

Fig. 2

Fig. 1

0 000 068

FIG. 3

FIG. 8

81

82

83

Z<sub>a</sub>

2.5 μ sec.

5.0 μsec.

FIG. 4A

81

82

83

Z<sub>a</sub>

Z<sub>b</sub>

Z<sub>c</sub>

0.5 μsec.

1.5 μsec.

FIG. 4B

FIG. 5

FIG. 6A

$D_1 = t_2$
$D_2 = t_2 - t_1$

FIG. 6B

FIG. 7